# EUROPEAN PATENT APPLICATION

(11) **EP 4 111 862 A1**
(43) Date of publication of application: **04.01.2023**
(21) Application number: 21761942.8
(22) Date of filing: 01.03.2021
(51) Int. Cl.: A01N 37/36, A01G 7/00, A01N 25/02, A01N 37/02, A01P 21/00, C05F 17/20

(54) **PLANT HEAT RESISTANCE OR DROUGHT RESISTANCE IMPROVING AGENT, SALT TOLERANCE IMPROVING AGENT, ACTIVITY IMPROVING AGENT**

(30) Priority: 28.02.2020 JP 2020034089
(71) Applicant: AC-Planta Inc., Tokyo 113-0034 (JP)
(72) Inventor: KIM, Jongmyong, Tokyo 113-0034 (JP)
(74) Representative: Hoffmann Eitle
(86) International application number: PCT/JP2021/007774
(87) International publication number: WO 2021/172594

(57) **Abstract**

The present invention relates to a heat tolerance or drying tolerance improving agent, a salt tolerance improving agent, or an activity improving agent for plants, comprising acetic acid or a salt thereof, or a solvate thereof and malic acid or a salt thereof, or a solvate thereof.

## Description

### Technical Field

The present invention relates to a heat tolerance or drying tolerance improving agent, a salt tolerance improving agent, an activity improving agent for plants.

### Background Art

As the population is now exploding, the production of plants serving as food in sufficient supplies is a matter of world concern. Also, the desertification of land ascribable to decrease in green space, etc. is problematic. In addition, for example, a rise in temperatures at the Earth's surface based on global warming phenomena is also problematic. The number of environmental problems associated with plant growth is increasing.

Specifically, excessive stresses on plants due to global environmental factors are often problematic to the growth of the plants.

One of the stresses on plants is drying stress.

To cope with the drying stress, attempts have been made on the development of genetically recombinant plants by engineering drying stress responsive genes or the application of chemical or biological regulating agents improving drying stress tolerance.

Patent Literature 1 discloses a method for improving the drying stress tolerance of a plant, comprising the step of applying 10 mM or higher acetic acid to the root of the plant by irrigation, and allowing the plant to grow under drying stress conditions.

Non Patent Literature 1 discloses a drought response network in which a plant acquires drying tolerance by the stimulation of a jasmonic acid signaling pathway which triggers the dynamic conversion of a metabolic flux from a glycolytic system to acetic acid synthesis.

### Citation List

### Patent Literature

Patent Literature 1: U.S. Patent No. 9,258,954

### Non Patent Literature

Non Patent Literature 1: Kim, J. M. et al., Nature Plants, Vol. 3, 17097 (2017)

### Summary of Invention

### Technical Problem

If the ability of plants to tolerate drying stress can be more enhanced, this is advantageous for plant growth. Accordingly, the present inventor has conducted studies by using whether the drying tolerance of plants can be further improved as an index.

There exist stresses other than drying stress as stresses on plants in plant growth. Accordingly, the present inventor has also conducted studies by using whether tolerance to stresses during plant growth in a high-temperature environment can be imparted to plants as an index.

An object of the present invention is to provide an approach that can impart heat tolerance or drying tolerance, salt tolerance, or activity to plants.

### Solution to Problem

The present inventor has conducted diligent studies to attain the object and has consequently completed the present invention by finding that the application of both acetic acid and malic acid contributes to the ability of a plant to grow favorably against heat stress and/or drying stress.

Specifically, the present invention is as follows.
(1) A heat tolerance or drying tolerance improving agent, a salt tolerance improving agent, or an activity improving agent for plants, comprising acetic acid or a salt thereof, or a solvate thereof and malic acid or a salt thereof, or a solvate thereof.
(2) A heat tolerance or drying tolerance improving agent, a salt tolerance improving agent, or an activity improving agent for plants, comprising acetic acid or a salt thereof, or a solvate thereof, for use in combination with malic acid or a salt thereof, or a solvate thereof.
(3) A heat tolerance or drying tolerance improving agent, a salt tolerance improving agent, or an activity improving agent for plants, comprising malic acid or a salt thereof, or a solvate thereof, for use in combination with acetic acid or a salt thereof, or a solvate thereof.
(4) The heat tolerance or drying tolerance improving agent, the salt tolerance improving agent, or the activity improving agent according to any of (1) to (3), further comprising one or more solvents including at least water.
(5) The heat tolerance or drying tolerance improving agent, the salt tolerance improving agent, or the activity improving agent according to (4), wherein a pH is in the range of 3 to 9.
(6) A composition for improving heat tolerance or drying tolerance, salt tolerance, or activity of a plant, comprising acetic acid or a salt thereof, or a solvate thereof and malic acid or a salt thereof, or a solvate thereof.
(7) A composition for improving heat tolerance or drying tolerance, salt tolerance, or activity of a plant, comprising acetic acid or a salt thereof, or a solvate thereof, for use in combination with malic acid or a salt thereof, or a solvate thereof.
(8) A composition for improving heat tolerance or drying tolerance, salt tolerance, or activity of a plant, comprising malic acid or a salt thereof, or a solvate thereof, for use in combination with acetic acid or a salt thereof, or a solvate thereof.
(9) The composition according to any of (6) to (8), further comprising one or more solvents including at least water.
(10) The composition according to (9), wherein a pH is in the range of 3 to 9.
(11) A method for improving heat tolerance or drying tolerance, salt tolerance, or activity of a plant, comprising applying acetic acid or a salt thereof, or a solvate thereof and malic acid or a salt thereof, or a solvate thereof to the plant, a material for application to the plant, or soil, a medium, or a culture solution where the plant grows.
(12) A method for controlling growth of a plant, comprising:
   obtaining one or more pieces of information on the growth of the plant; and
   on the basis of the obtained one or more pieces of information, determining conditions under which the heat tolerance or drying tolerance improving agent, salt tolerance improving agent, or activity improving agent according to any of (1) to (5) or the composition according to any of (6) to (10) is applied to the plant, a material for application to the plant, or soil, a medium, or a culture solution where the plant grows.
(13) A method for improving heat tolerance or drying tolerance, salt tolerance, or activity of a plant, comprising using acetic acid or a salt thereof, or a solvate thereof and malic acid or a salt thereof, or a solvate thereof in combination.
(14) A method for improving heat tolerance or drying tolerance, salt tolerance, or activity of a plant, comprising using acetic acid or a salt thereof, or a solvate thereof and malic acid or a salt thereof, or a solvate thereof.

### Advantageous Effects of Invention

The present invention can provide an approach that can impart heat tolerance, drying tolerance or salt tolerance and/or activity to plants.

### Brief Description of Drawings

[Figure 1] Figures 1A and 1B are diagram showing the results of heat drying tolerance test 1 and heat drying tolerance test 2, respectively, using stem lettuce *(Lactuca sativa* L.).
[Figure 2] Figure 2 is a diagram showing the results of heat drying tolerance test 3 using tomato *(Solanum lycopersicum).*
[Figure 3] Figure 3 is a diagram showing the results of activation test 1 (effect on the induction and growth of flower buds) using peperomia *(Peperomia albovittata*)*.*
[Figure 4] Figure 4 is a diagram showing the results of activation test 2 (effect on plant growth (plant size after harvest)) using spinach *(Spinacia oleracea).*
[Figure 5] Figure 5 is a diagram showing the results of activation test 2 (effect on plant growth (plant size after harvest)) using spinach.
[Figure 6] Figure 6 is a diagram showing the results of activation test 3 (effect on root growth (plant size after harvest)) using carrot *(Daucus carota* subsp. *sativus).*

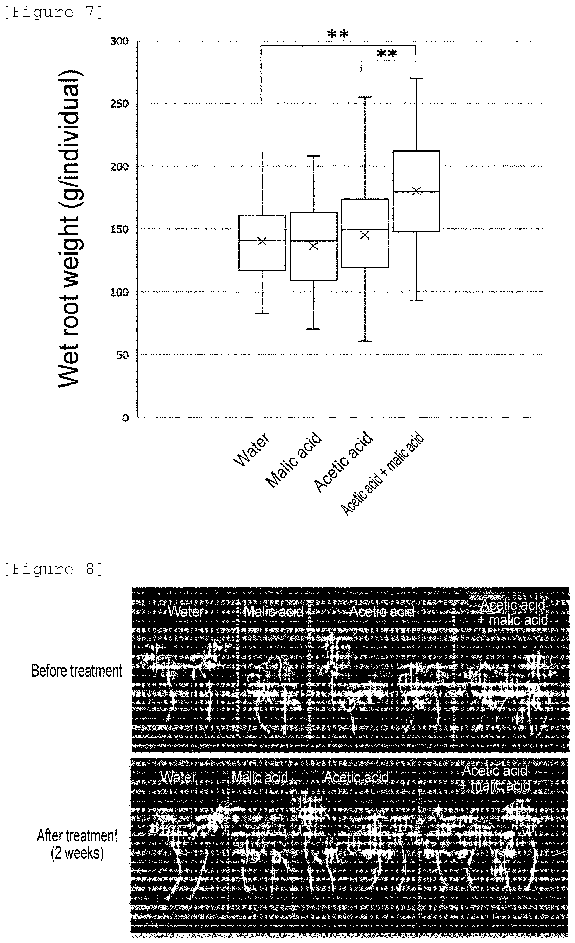

[Figure 7] Figure 7 is a diagram showing the results of activation test 3 (effect on root growth (plant size after harvest)) using carrot.
[Figure 8] Figure 8 is a diagram showing the results of activation test 4 (effect on the induction of roots) using aromaticus *(Plectranthus amboinicus*)*.*

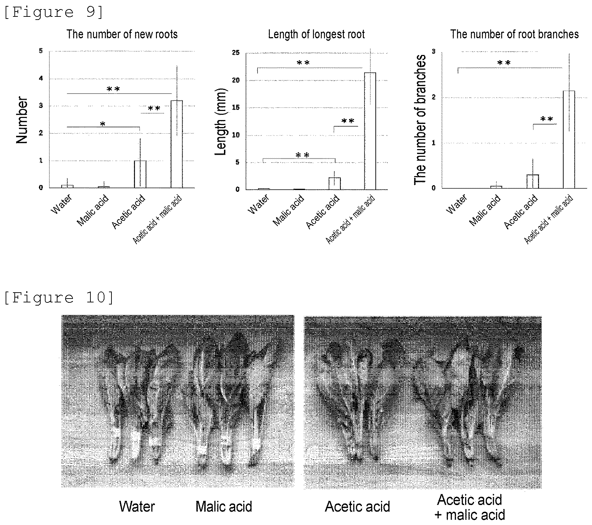

[Figure 9] Figure 9 is a diagram showing the results of activation test 4 (effect on the induction of roots) using aromaticus.
[Figure 10] Figure 10 is a diagram showing the results of activation test 5 (the maintenance of freshness and water after harvest) using spinach.

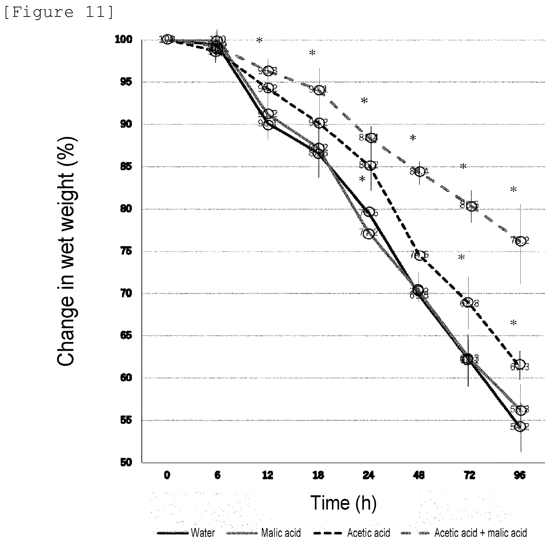

[Figure 11] Figure 11 is a diagram showing the results of activation test 5 (the maintenance of freshness and water after harvest) using spinach.

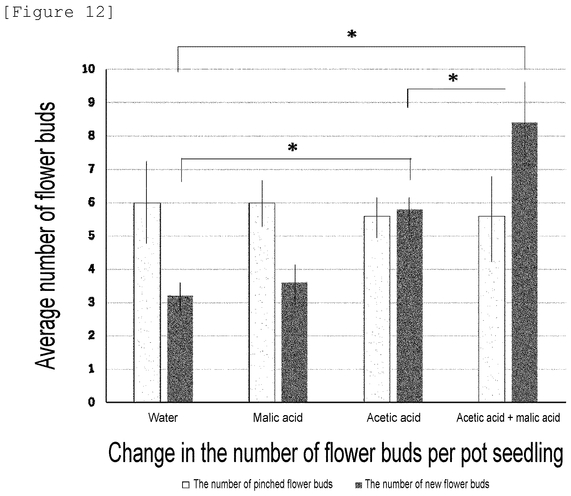

[Figure 12] Figure 12 is a diagram showing the results of activation test 6 (effect on the induction and growth of flower buds) using swamp chrysanthemum *(Leucanthemum paludosum).*

### Description of Embodiments

Hereinafter, the embodiments of the present invention will be described in detail. However, the present invention is not limited by the embodiments given below, and can be carried out with various changes or modifications made therein.

The present invention provides a heat tolerance or drying tolerance improving agent, a salt tolerance improving agent, or an activity improving agent for plants, comprising acetic acid or a salt thereof, or a solvate thereof and malic acid or a salt thereof, or a solvate thereof.

In the present invention, the heat tolerance and/or drying tolerance, salt tolerance, or activity of a plant can be improved by applying the heat tolerance or drying tolerance improving agent, the salt tolerance improving agent, or the activity improving agent for plants to the plant.

The heat tolerance or drying tolerance improving agent for plants of the present invention may be a heat tolerance improving agent, may be a drying tolerance improving agent, or may be both of the heat tolerance improving agent and the drying tolerance improving agent. The heat tolerance improving agent or the drying tolerance improving agent for plants according to the present invention may be a heat tolerance improving agent or a drought tolerance improving agent, etc. for plants. Furthermore, the heat tolerance improving agent or the drying tolerance improving agent for plants according to the present invention may be a heat drying tolerance improving agent when being an agent that can improve the heat tolerance and/or drying tolerance of a plant.

The heat tolerance or drying tolerance improving agent, the salt tolerance improving agent, or the activity improving agent for plants of the present invention can be used as an agricultural chemical or an agrochemical formulation.

In the present invention, tolerance to stresses during plant growth in a high-temperature environment or in an environment such as saline soil, which has unfavorable influence on the plant growth, can be imparted to a plant by applying the heat tolerance or drying tolerance improving agent, the salt tolerance improving agent, or the activity improving agent for plants to the plant. In the present specification, the imparting of tolerance to stresses during plant growth to the plant does not mean that complete tolerance is conferred.

In the present invention, the heat tolerance or drying tolerance improving agent, the salt tolerance improving agent, or the activity improving agent for plants comprises acetic acid or a salt thereof, or a solvate thereof (hereinafter, also referred to as "acetic acid, etc." in the present specification).

In the present invention, the heat tolerance or drying tolerance improving agent, the salt tolerance improving agent, or the activity improving agent for plants comprises malic acid or a salt thereof, or a solvate thereof (hereinafter, also referred to as "malic acid, etc." in the present specification).

In this context, the acetic acid, etc. is not particularly limited as long as the acetic acid, etc. exerts characteristics as the heat tolerance or drying tolerance improving agent, the salt tolerance improving agent, or the activity improving agent for plants, together with the malic acid, etc. The heat tolerance or drying tolerance improving agent, the salt tolerance improving agent, or the activity improving agent for plants contains the acetic acid, etc. as an active ingredient.

In the present invention, the active ingredient means that the ingredient contained in the heat tolerance or drying tolerance improving agent, the salt tolerance improving agent, or the activity improving agent for plants is an ingredient that can improve heat tolerance or drying tolerance, salt tolerance, or activity as characteristics as the agent.

The acetic acid, etc. used in the present invention may be acetic acid, etc. for industrial use or may be acetic acid, etc. for food use.

The acetic acid that may be used can be safe and inexpensive acetic acid such as pyroligneous acid which is also used for agricultural use, or fermented vinegar which is produced by fermentation or the like.

Use of such acetic acid, etc. as the heat tolerance or drying tolerance improving agent, the salt tolerance improving agent, or the activity improving agent for plants of the present invention is suitable because of high safety and low cost.

The acetic acid, etc. used in the present invention may not only be acetic acid itself but may be a salt of acetic acid.

The salt of acetic acid is not particularly limited as long as the salt is capable of supplying an acetate ion. Examples thereof include salts with cations and specifically include sodium acetate, potassium acetate, calcium acetate, magnesium acetate, zinc acetate ions, and ammonium acetate.

The ammonium acetate may be acetate with substituted or unsubstituted ammonium instead of an ammonium ion.

Among them, potassium acetate, sodium acetate, ammonium acetate, magnesium acetate, or calcium acetate can be suitably used as the salt of acetic acid. Alternatively, a liquid fertilizer obtained by dissolving an eggshell in vinegar may be used as the acetic acid, etc.

In the present invention, acetic acid and acetate may be used as a mixture. The mixture of acetic acid and acetate may be a mixture of acetic acid itself with acetate or may be a mixed solution of acetic acid and acetate formed, for example, as an aqueous solution through neutralization reaction by adding a cation source that forms a salt with an acetate ion to the acetic acid. In this context, the acetic acid may be completely neutralized, or the cation source may be added in an amount that allows the acetic acid to be partially neutralized.

Examples of the aqueous solution containing acetate include acetate buffer solutions.

The acetic acid, etc. used in the present invention may be a solvate of acetic acid or a solvate of acetate.

Examples of the solvent capable of forming the solvate of the acetic acid or a salt thereof include, but are not particularly limited to, water, and organic solvents such as alcohols, dimethyl sulfoxide (DMSO), ethanolamine and ethyl acetate.

The alcohol may be a lower alcohol or may be a higher alcohol, for example. Examples of the lower alcohol include, but are not particularly limited to, saturated or unsaturated linear or branched alkyl alcohols having 1 to 6 carbon atoms, such as methanol, ethanol and 2-propanol (isopropyl alcohol). Examples of the higher alcohol include, but are not particularly limited to, saturated or unsaturated linear or branched alkyl alcohols having 7 or more carbon atoms, such as 1-heptanol and 1-octanol.

These solvents forming the solvate may each be used singly or may be two or more solvents.

In the case of using the solvate, for example, as an aqueous solution of the solvate, the acetic acid, etc. in the aqueous solution is not particularly limited by its form and may not be solvated.

The acetic acid, etc. used in the present invention is acetic acid or a salt thereof, or a solvate thereof and can be one or two or more compounds selected from the group consisting of acetic acid and a salt thereof, and a solvate thereof. The compounds included in the group consisting of acetic acid and a salt thereof, and a solvate thereof can be appropriately selected in an arbitrary combination from the aforementioned compounds described about acetic acid, the salt of acetic acid, or the solvate thereof.

In the agent, the composition, etc. of the present invention, the acetic acid, etc. may exist as acetic acid and/or a salt of acetic acid. The salt of acetic acid can be potassium acetate, sodium acetate, ammonium acetate, magnesium acetate, or calcium acetate and may be selected from an arbitrary combination thereof. The salt of acetic acid may be potassium acetate, sodium acetate, and/or ammonium acetate, may be potassium acetate and/or sodium acetate, or may be magnesium acetate and/or calcium acetate.

In the present invention, the heat tolerance or drying tolerance improving agent, the salt tolerance improving agent, or the activity improving agent for plants comprises malic acid, etc. The malic acid, etc. is not particularly limited as long as the malic acid, etc. exerts characteristics as the heat tolerance or drying tolerance improving agent, the salt tolerance improving agent, or the activity improving agent for plants. The heat tolerance or drying tolerance improving agent, the salt tolerance improving agent, or the activity improving agent for plants contains the malic acid, etc. as an active ingredient.

The malic acid, etc. used in the present invention may be malic acid, etc. that may be used for every purpose, or may be malic acid, etc. for industrial use or for food use, without particular limitations.

Use of such malic acid, etc. as the heat tolerance or drying tolerance improving agent, the salt tolerance improving agent, or the activity improving agent for plants of the present invention is suitable because of high safety and low cost.

The malic acid, etc. used in the present invention may not only be malic acid itself but may be a salt of malic acid.

The salt of malic acid is not particularly limited as long as the salt is capable of supplying a malate ion. Examples thereof include salts with cations and specifically include sodium malate, potassium malate, calcium malate, magnesium malate, zinc malate ions, and ammonium malate.

The ammonium malate may be malate with substituted or unsubstituted ammonium instead of an ammonium ion.

Among them, potassium malate, sodium malate, ammonium malate, magnesium malate, or calcium malate can be suitably used as the salt of malic acid. Alternatively, a liquid fertilizer obtained by dissolving an eggshell in apple vinegar may be used as the malic acid, etc.

In the present invention, malic acid and malate may be used as a mixture. The mixture of malic acid and malate may be a mixture of malic acid itself with malate or may be a mixed solution of malic acid and malate formed, for example, as an aqueous solution through neutralization reaction by adding a cation source that forms a salt with a malate ion to the malic acid. In this context, the malic acid may be completely neutralized, or the cation source may be added in an amount that allows the malic acid to be partially neutralized.

Examples of the aqueous solution containing malate include malate buffer solutions.

The malic acid, etc. used in the present invention may be a solvate of malic acid or a solvate of malate.

Examples of the solvent capable of forming the solvate of the malic acid or a salt thereof include, but are not particularly limited to, water, and organic solvents such as alcohols, dimethyl sulfoxide (DMSO), ethanolamine and ethyl acetate.

The alcohol may be a lower alcohol or may be a higher alcohol, for example. Examples of the lower alcohol include, but are not particularly limited to, saturated or unsaturated linear or branched alkyl alcohols having 1 to 6 carbon atoms, such as methanol, ethanol and 2-propanol (isopropyl alcohol). Examples of the higher alcohol include, but are not particularly limited to, saturated or unsaturated linear or branched alkyl alcohols having 7 or more carbon atoms, such as 1-heptanol and 1-octanol.

These solvents forming the solvate may each be used singly or may be two or more solvents.

In the case of using the solvate, for example, as an aqueous solution of the solvate, the malic acid, etc. in the aqueous solution is not particularly limited by its form and may not be solvated.

The malic acid, etc. used in the present invention is malic acid or a salt thereof, or a solvate thereof and can be one or two or more compounds selected from the group consisting of malic acid and a salt thereof, and a solvate thereof. The compounds included in the group consisting of malic acid and a salt thereof, and a solvate thereof can be appropriately selected in an arbitrary combination from the aforementioned compounds described about malic acid, the salt of malic acid, or the solvate thereof.

In the agent, the composition, etc. of the present invention, the malic acid, etc. may exist as malic acid and/or a salt of malic acid. The salt of malic acid can be potassium malate, sodium malate, ammonium malate, magnesium malate, or calcium malate and may be selected from an arbitrary combination thereof. The salt of malic acid may be potassium malate, sodium malate, and/or ammonium malate, may be potassium malate and/or sodium malate, or may be magnesium malate and/or calcium malate.

The salt of acetic acid and the salt of malic acid may be derived from the same cation source. In the case of using different cation sources for these salts, respectively, the cation sources may exist in salt forms (which, however, are not recognized as specific salts, if dissociated) with acetic acid and malic acid at their respective ratios according to their properties, for example, in a solution (preferably an aqueous solution).

The heat tolerance improving agent or the drying tolerance improving agent, the salt tolerance improving agent, or the activity improving agent according to the present invention may contain acetic acid, etc. for use in combination with malic acid, etc. or may contain malic acid, etc. for use in combination with acetic acid, etc.

The use of acetic acid, etc. and malic acid, etc. in combination may be the application of the acetic acid, etc. and the malic acid, etc. as one mixture to a plant, or may be the application of a composition comprising the acetic acid, etc. and a composition comprising the malic acid, etc. at the same time or at different times to a plant. The acetic acid, etc. may be applied to a plant and thereby expected to exert the desired effect by use in combination with malic acid, etc. present in the plant, or the malic acid, etc. may be applied to a plant and thereby expected to exert the desired effect by use in combination with acetic acid, etc. present in the plant.

In the present invention, the term "heat tolerance improving agent" or "improving heat tolerance" means that the application of the heat tolerance improving agent for plants of the present invention to a plant population can substantially reduce heat stress, which has unfavorable influence on plant growth, such as inability to grow (firing), poor growth (e.g., the whitening or yellowing of the whole plant or a portion thereof (e.g., leaves or flowers), decrease in root length or decrease in the number of leaves, or lodging), decrease in growth rate, or decrease in plant weight or crop yield.

Being the "heat tolerance improving agent" or "improving heat tolerance" can be confirmed by comparison with a control plant population to which the heat tolerance improving agent for plants of the present invention is not applied, and can usually be confirmed from 30% or more, preferably 50% or more, 60% or more, or 70% or more, more preferably 80% or more, further preferably 85% or more, particularly preferably 90% or more improvement in survival rate.

In the present specification, the "heat stress" means being left in an environment having a temperature of 60°C or lower. The temperature may be 50°C or lower in the environment or may be 45°C or lower in the environment.

Usually, considering that a constant temperature is on the order of 25°C, the "heat stress" is preferably an environment of 30°C or higher, more preferably an environment of 35°C or higher.

In the present invention, the confirmation of being the "heat tolerance improving agent" or "improving heat tolerance" is not particularly limited. This may be evaluated by the following approach and more specifically, may be evaluated by a method described in Examples.

For example, targeted plants are allowed to grow under usual growth conditions, i.e., under non-heat-stress conditions, in a given amount of a test solution (containing water and optionally a usual nutrient component). After growth for a given period, the heat tolerance improving agent is applied to the plants, which are then allowed to grow under heat stress conditions and subsequently grow under usual growth conditions, i.e., under non-heat-stress conditions. The survival rate of the targeted plants can be measured for evaluation.

The heat stress conditions can be conditions involving still standing for 30 minutes or longer under the temperature conditions of the "heat stress". The time of still standing may be set according to the temperature conditions.

The heat stress conditions are preferably conditions involving still standing without particular water supply under constant humidity conditions.

In the present invention, the term "drying tolerance improving agent" or "improving drying tolerance" means that the application of the drying tolerance improving agent for plants of the present invention to a plant population can substantially reduce drying stress, which has unfavorable influence on plant growth, such as inability to grow (firing), poor growth (e.g., the whitening or yellowing of the whole plant or a portion thereof (e.g., leaves or flowers), decrease in root length or decrease in the number of leaves, or lodging), decrease in growth rate, or decrease in plant weight or crop yield.

Being the "drying tolerance improving agent" or "improving drying tolerance" can be confirmed by comparison with a control plant population to which the drying tolerance improving agent for plants of the present invention is not applied, and can usually be confirmed from 30% or more, preferably 50% or more, 60% or more, or 70% or more, more preferably 80% or more, further preferably 85% or more, particularly preferably 90% or more improvement in survival rate.

In the present specification, the "drying stress" means being left in an environment having a low humidity.

The cause of the drying stress is not particularly limited and, in the present invention, can be drying stress based on temperature conditions. Examples thereof include drying stress associated with a high temperature without the addition of water.

Change in humidity influences change in the water contents of a plant individual and soil. Thus, the effect of improving heat tolerance or drying tolerance is more exerted at a high humidity. Also, the effect of the mixed malic acid, etc. is more exerted under gradual heat or drying stress at a high humidity. Specifically, such an effect is more exerted at a humidity that facilitates heat and/or drying stress.

In the present invention, the confirmation of being the "drying tolerance improving agent" or "improving drying tolerance" is not particularly limited. This may be evaluated by the following approach and more specifically, may be evaluated by a method described in Examples.

For example, targeted plants are allowed to grow under usual growth conditions, i.e., under non-drying-stress conditions, in an environment containing a given amount of a test solution (water and optionally a usual nutrient component). After growth for a given period, the drying tolerance improving agent is applied to the plants, which are then allowed to grow under drying stress conditions and subsequently grow under usual growth conditions, i.e., under non-drying-stress conditions. The survival rate of the targeted plants can be measured for evaluation.

The drying stress conditions can be conditions involving still standing for 30 minutes or longer under the humidity conditions of the "drying stress". The time of still standing may be set according to the type or concentration conditions of the drying tolerance improving agent.

The drying stress conditions are preferably conditions involving still standing without particular water supply under low-humidity conditions.

In the present invention, the heat tolerance or drying tolerance improving agent for plants may be a heat tolerance improving agent for plants, may be a drying tolerance improving agent for plants, or may be the heat tolerance improving agent for plants while being the drying tolerance improving agent for plants.

In the present invention, the heat tolerance or drying tolerance improving agent, the salt tolerance improving agent for plants may be a composition for improving heat tolerance or drying tolerance, salt tolerance, or activity of a plant, the composition comprising the acetic acid, etc. and the malic acid, etc.

Specifically, in the present specification, the "heat tolerance or drying tolerance improving agent, the salt tolerance improving agent, or the activity improving agent for plants" may be interpreted as having the same meaning as the "composition for improving heat tolerance or drying tolerance, salt tolerance, or activity of a plant".

In each aspect of the present invention, the effect of the heat tolerance or drying tolerance improving agent, the salt tolerance improving agent, or the activity improving agent for plants may include an effect on the growth itself of a plant, for example, a growth promoting effect such as the elongation of leaves and stems or roots, increase in the number of leaves, the promotion of flowering or fructification, increase in the number of flowers or fruits, increase in plant weight or crop yield, greening, or the promotion of tillering.

The heat tolerance or drying tolerance improving agent for plants of the present invention may be used for the activation of plants as described below through the use of or without the use of the property of heat tolerance or drying tolerance.

The present invention may provide an activity improving agent for plants, comprising acetic acid, etc. and malic acid, etc., wherein the activity improving agent exhibits any of the following effects:
1) the elongation of roots and improvement in the survival rate of roots,
2) increase in nutrient absorption efficiency,
3) the induction of flower buds, the promotion of flowering, and increase in the amount of fructification,
4) the accumulation of components such as sugar,
5) enhancement in water saving effect,
6) callus formation inducing effect,
7) improvement in plant size, particularly, the elongation or enlargement of aerial parts and underground parts, and
8) wound repair.

In the present invention, the activity improving agent for plants may exhibit any of these effects 1) to 8) or may have a growth promoting effect such as the elongation of leaves and stems or roots, increase in the number of leaves, the promotion of flowering or fructification, increase in the number of flowers or fruits, increase in plant weight or crop yield, greening, or the promotion of tillering.

Specifically, in the present invention, the acetic acid, etc. and the malic acid, etc. may be used as an activating agent or a vitality enhancing agent for plants by application to the plants.

The activating agent or the vitality enhancing agent is meant to have advantages in the growth of plants. In the present invention, vitality can be imparted to a plant by applying acetic acid, etc. and malic acid, etc. to the plant. In the present invention, the growth of a plant can be promoted by applying acetic acid, etc. and malic acid, etc. to the plant. Thus, the acetic acid, etc. and the malic acid, etc. may be used as a growth promoting agent.

The present invention may provide a salt tolerance improving agent for plants, comprising acetic acid, etc. and malic acid, etc.

In the present invention, heat tolerance and/or drying tolerance to heat stress and/or drying stress can be improved by applying acetic acid, etc. and malic acid, etc. to a plant.

In the present invention, salt tolerance to salt stress can be improved by applying acetic acid, etc. and malic acid, etc. to a plant.

In the present invention, plant activity can be improved by applying acetic acid, etc. and malic acid, etc. to a plant.

The heat tolerance or drying tolerance improving agent, the salt tolerance improving agent, or the activity improving agent for plants or the composition for improving the heat tolerance or drying tolerance, salt tolerance, or activity of a plant according to the present invention can be used as an agrochemical formulation or an agricultural chemical for promoting plant growth.

In the present invention, the heat tolerance or drying tolerance improving agent, the salt tolerance improving agent, or the activity improving agent for plants may be in an arbitrary form, for example, a solid (e.g., a powder or grains), a liquid (e.g., a solution or a suspension), or a gas.

In the present invention, the heat tolerance or drying tolerance improving agent, the salt tolerance improving agent, or the activity improving agent for plants is preferably used in the form of a liquid such as a solution or a suspension.

In the present invention, in the case of using the heat tolerance or drying tolerance improving agent, the salt tolerance improving agent, or the activity improving agent for plants as a solution, the solution may be in a liquid state or may be used as a liquid prepared just before use for application.

In the present invention, the contents described about the heat tolerance or drying tolerance improving agent for plants of the present invention are also applicable to use as a heat drying tolerance improving agent. The same holds true for use as a salt tolerance improving agent for plants or an activity improving agent for plants.

In the heat tolerance or drying tolerance improving agent, the salt tolerance improving agent, or the activity improving agent for plants of the present invention, the acetic acid, etc. and the malic acid, etc., preferably, acetic acid and malic acid, may be used singly as an active ingredient or may be used in combination with one or more agriculturally acceptable components.

The acetic acid, etc. and malic acid may be used, or acetic acid and the malic acid, etc. may be used. One or two or more of the acetic acid, etc. may be used, and one or two or more of the malic acid, etc. may be used. Specifically, two or more of the acetic acid, etc. may be used; two or more of the malic acid, etc. may be used; acetic acid and one or more of the acetic acid, etc. except for acetic acid and malic acid and one or more of the malic acid, etc. except for malic acid may be used; acetic acid, malic acid, and one or more of the malic acid, etc. except for malic acid may be used; acetic acid, one or more of the acetic acid, etc. except for acetic acid, and malic acid may be used; and acetic acid and malic acid may be used. Alternatively, one or more of the acetic acid, etc. except for acetic acid and one or more of the malic acid, etc. except for malic acid may be used.

The heat tolerance or drying tolerance improving agent, the salt tolerance improving agent, or the activity improving agent for plants of the present invention can be formulated as an agricultural chemical or an agrochemical formulation into various dosage forms usually used in the art according to the desired application method.

The heat tolerance or drying tolerance improving agent, the salt tolerance improving agent, or the activity improving agent for plants of the present invention may further contain one or more agriculturally acceptable components, in addition to the acetic acid, etc. and the malic acid, etc.

Examples of the agriculturally acceptable component includes solvents or carriers, excipients, binders, solubilizers, stabilizers, thickeners, puffing agents, lubricants, surfactants, oily liquids, buffers, germicides, antifreezes, antifoaming agents, colorants, antioxidants, additives, fertilizers, and other agents.

The agriculturally acceptable solvent or carrier is preferably an agriculturally acceptable solvent or liquid carrier such as water, a mineral oil fraction such as kerosene or diesel fuel, a plant- or animal-derived oil, cyclic or aromatic hydrocarbon (e.g., paraffin, tetrahydronaphthalene, alkylated naphthalenes or their derivatives, or alkylated benzenes or their derivatives), an alcohol (e.g., methanol, ethanol, propanol, butanol, ethylene glycol, glycerol or cyclohexanol), ketone (e.g., cyclohexanone), or amine (e.g., N-methylpyrrolidone), or a mixture thereof, more preferably one or more solvents including at least water.

The fertilizer is preferably an organic fertilizer such as oil cake or bovine feces, or an inorganic fertilizer such as ammonium sulfate, lime nitrogen or fused phosphate.

When the heat tolerance or drying tolerance improving agent, the salt tolerance improving agent, or the activity improving agent for plants of the present invention further contains one or more solvents including at least water, the pH of the solution of the heat tolerance or drying tolerance improving agent, the salt tolerance improving agent, or the activity improving agent for plants of the present invention is preferably in the range of 3 to 9. Within this range, the lower limit value of the pH may be 4 or higher, 5 or higher, or 6 or higher, and the upper limit value of the pH may be 8.5 or lower, 8 or lower, 7.5 or lower, or 7 or lower.

The range of the pH is more preferably the range of 4 to 8, further preferably the range of 5 to 7.5, still further preferably the range of 5 to 7. The range of the pH may be 5 to 6 or may be 6 to 7.

The pH of the heat tolerance or drying tolerance improving agent, the salt tolerance improving agent, or the activity improving agent for plants preferably falls within the above range at the point in time when the heat tolerance or drying tolerance improving agent, the salt tolerance improving agent, or the activity improving agent for plants is applied to a target plant.

The pH of the heat tolerance or drying tolerance improving agent, the salt tolerance improving agent, or the activity improving agent for plants of the present invention may be adjusted with an acid or an alkali and may be adjusted using, for example, an acid, an alkali or a buffer, such as hydrochloric acid, nitric acid, sulfuric acid, sodium hydroxide, potassium hydroxide, ammonia water or ammonium acetate.

For the heat tolerance or drying tolerance improving agent, the salt tolerance improving agent, or the activity improving agent for plants of the present invention, its pH preferably falls within the above range at the point in time when the heat tolerance or drying tolerance improving agent, the salt tolerance improving agent, or the activity improving agent for plants is applied to a target plant. However, even if the pH falls outside the above range at the point in time when the heat tolerance or drying tolerance improving agent, the salt tolerance improving agent, or the activity improving agent for plants is applied to a target plant, the pH may be adjusted to within the above range through the use of the pH buffering effect of soil, a medium or a culture solution for application.

When the heat tolerance or drying tolerance improving agent, the salt tolerance improving agent for plants of the present invention further contains one or more solvents including at least water, the proportion of the acetic acid, etc. to the total volume in the heat tolerance or drying tolerance improving agent, the salt tolerance improving agent for plants of the present invention is preferably in the range of 0.01 to 0.5% by volume. Within this range, the lower limit value of the proportion may be 0.05% by volume or more, 0.075% by volume or more, 0.09% by volume or more, or 0.1% by volume or more, and the upper limit value of the proportion may be 0.25% by volume or less or 0.2% by volume or less.

The range of the proportion of the acetic acid, etc. is more preferably the range of 0.05 to 0.5% by volume, further preferably the range of 0.075 to 0.25% by volume, still further preferably the range of 0.09 to 0.2% by volume, particularly preferably the range of 0.1 to 0.2% by volume.

When the heat tolerance or drying tolerance improving agent, the salt tolerance improving agent for plants of the present invention further contains one or more solvents including at least water, the concentration of the acetic acid, etc. in the heat tolerance or drying tolerance improving agent, the salt tolerance improving agent for plants of the present invention is preferably in the range of 1 to 100 mM. Within this range, the lower limit value of the concentration may be 2 mM or higher, 5 mM or higher, 7.5 mM or higher, 9 mM or higher, or 10 mM or higher, and the upper limit value of the concentration may be 50 mM or lower, 40 mM or lower or 30 mM or lower.

The range of the proportion of the acetic acid, etc. is more preferably the range of 1 to 50 mM, further preferably the range of 7.5 to 50 mM, still further preferably the range of 9 to 40 mM, particularly preferably the range of 10 to 40 mM.

When the heat tolerance or drying tolerance improving agent or the salt tolerance improving agent for plants of the present invention further contains one or more solvents including at least water, the concentration of the malic acid, etc. in the heat tolerance or drying tolerance improving agent or the salt tolerance improving agent for plants of the present invention is preferably in the range of 1 to 100 mM. Within this range, the lower limit value of the concentration may be 2 mM or higher, 5 mM or higher, 7.5 mM or higher, 9 mM or higher, or 10 mM or higher, and the upper limit value of the concentration may be 50 mM or lower, 40 mM or lower, or 30 mM or lower.

The range of the proportion of the malic acid, etc. is more preferably the range of 1 to 50 mM, further preferably the range of 7.5 to 50 mM, still further preferably the range of 9 to 40 mM, particularly preferably the range of 10 to 40 mM.

In the case of using the acetic acid, etc. and the malic acid, etc. according to the present invention in combination or as a mixture for plant activation, the concentration of the malic acid, etc. may be in the range of 1 to 100 mM or may be in the range of 1 mM or lower, in addition to the contents described about the concentrations (% by volume or mM) of the acetic acid, etc. and the malic acid, etc. for use as the heat tolerance or drying tolerance improving agent or the salt tolerance improving agent for plants. For use in plant activation, the concentration of the malic acid, etc. may be in the range of higher than 0 mM to 100 mM, and the lower limit value may be set within the range of higher than 0 mM and 1 mM or lower or may be the lower limit value described above within the range of 1 mM or higher. The lower limit value of the concentration within the range of 1 mM or lower may be the lower limit value of a concentration on the order of 10⁻⁵, a concentration on the order of 10⁻⁴, a concentration on the order of 10⁻³, a concentration on the order of 10⁻², or a concentration on the order of 10⁻¹, in terms of mM.

When the heat tolerance or drying tolerance improving agent, the salt tolerance improving agent, or the activity improving agent for plants of the present invention further contains one or more solvents including at least water, preferably, the pH falls within the above range and the concentrations of the acetic acid, etc. and the malic acid, etc. fall within the above ranges.

The pH and the concentrations can be appropriately selected within the above ranges.

The heat tolerance or drying tolerance improving agent, the salt tolerance improving agent, or the activity improving agent for plants of the present invention may further contain one or more additional agents.

Examples of the agent include, but are not particularly limited to, auxin, gibberellin, cytokinin, 2-chloroethylphosphonic acid (trade name: ESREL(R)), carbide, benzyladenine, brassinosteroid, strigolactone and jasmonic acid. The agent may be a phytohormone, a plant chemical regulating agent and an agricultural chemical, etc. usually used in the art.

In the present invention, the targeted plant is not particularly limited and is selected from, for example, an angiosperm and a gymnosperm.

Examples of the targeted plant include, but are not particularly limited to, plants of the family *Compositae* such as florists' daisy *(Chrysanthemum morifolium)* and gerbera *(Gerbera jamesonii),* plants of the family *Solanaceae* such as potato *(Solanum tuberosum),* tomato *(Solanum lycopersicum)* and eggplant *(Solanum melongena),* plants of the family *Brassicaceae* such as rape *(Brassica napus* L.) and turnip rape *(Brassica rapa* L. var. *nippo-oleifera),* plants of the family *Poaceae* such as rice *(Oryza sativa),* corn *(Zea mays),* wheat *(Triticum* L.), sugar cane *(Saccharum officinarum)* and barley *(Hordeum vulgare),* plants of the family *Fabaceae* such as soybean *(Glycine max),* plants of the family *Apiaceae* such as carrot *(Daucus carota* subsp. *sativus),* plants of the family *Lamiaceae* such as basil *(Ocimum basilicum),* mint *(Mentha* L.), and rosemary *(Salvia rosmarinus),* plants of the family *Convolvulaceae* such as morning glory *(Ipomoea nil),* plants of the family *Salicaceae* such as poplar *(Populus nigra* var. *italica),* plants of the family *Euphorbiaceae* such as castor-oil plant *(Ricinus communis),* cassava *(Manihot esculenta)* and Barbados nut *(Jatropha curcas),* plants of the family *Convolvulaceae* such as sweet potato *(Ipomoea batatas),* plants of the family *Rutaceae* such as orange *(Citrus sinensis)* and lemon *(Citrus limon),* plants of the family *Rosaceae* such as Japanese cherry *(Prunus lannesiana* cv.) and rose *(Rosa* sp.), plants of the family *Orchidaceae* such as orchid *(Phalaenopsis aphrodite),* plants of the family *Gentianaceae* such as showy prairie gentian *(Eustoma grandiflorum*)*,* plants of the family *Primulaceae* such as cyclamen *(Cyclamen persicum* cv.), plants of the family *Violaceae* such as pansy *(Viola × wittrockiana),* plants of the family *Liliaceae* such as lily *(Lilium* L.), plants of the family *Amaranthaceae* such as sugar beet *(Beta vulgaris* ssp. *vulgaris),* plants of the family *Vitaceae* such as grape *(Vitis* spp.), plants of the family *Cupressaceae* such as cryptomeria *(Cryptomeria japonica)* and hinoki cypress *(Chamaecyparis obtusa),* plants of the family *Oleaceae* such as olive *(Olea europaea)* and sweet tea *(Osmanthus fragrans* var. *aurantiacus),* and plants of the family *Pinaceae* such as Japanese red pine *(Pinus densiflora).* Such a plant may be a plant used in Examples.

The targeted plant may not only be the whole plant (i.e., the whole body of the plant) but may be a portion of the plant, such as a plant tissue or organ (e.g., cut flowers, or vegetative reproductive organs such as rhizomes, tuberous roots, corms or runners), cultured cells and/or calluses.

The heat tolerance or drying tolerance improving agent, the salt tolerance improving agent, or the activity improving agent for plants of the present invention can be applied to the whole plant or a portion thereof (e.g., seeds, seedlings or the whole mature plant or a portion thereof) at an arbitrary stage of growth including before and after budding of the plant.

The heat tolerance or drying tolerance improving agent, the salt tolerance improving agent, or the activity improving agent for plants of the present invention may be applied not only to a plant itself but to a material for application to the plant, or soil, a medium, or a culture solution where the plant grows.

The present invention also relates to a method for improving heat tolerance or drying tolerance, salt tolerance, or activity of a plant, comprising applying the acetic acid, etc. and the malic acid, etc., preferably an agriculturally effective amount of the acetic acid and the malic acid to the plant, a material for application to the plant, or soil, a medium, or a culture solution where the plant grows. In this case, the acetic acid, etc. and the malic acid, etc. may be applied preferably as a mixed solution containing agriculturally effective amounts of acetic acid and malic acid.

In the method of the present invention, the acetic acid, etc., the malic acid, etc., to be applied and other aspects are as described about the heat tolerance or drying tolerance improving agent, the salt tolerance improving agent, or the activity improving agent for plants in the present specification.

Examples of the material for application to the plant include, but are not particularly limited to, various materials usually used in the art, such as water and fertilizers.

The dosage form of the heat tolerance or drying tolerance improving agent, the salt tolerance improving agent, or the activity improving agent for plants of the present invention is not particularly limited and can be a dosage form, such as an emulsion, a wettable powder, a solution, an aqueous solution, a dust, a powder, a paste, or granules, usually used in the art.

In each aspect of the present invention, the acetic acid, etc. is contained or applied in an agriculturally effective amount. In each aspect of the present invention, the agriculturally effective amount of the acetic acid, etc. is, for example, in the range of 0.01 to 0.5% by mass with respect to the total mass at the time of application, usually in the range of 0.05 to 0.5% by mass with respect to the total mass at the time of application, typically in the range of 0.075 to 0.25% by mass with respect to the total mass at the time of application, more typically in the range of 0.09 to 0.2% by mass with respect to the total mass at the time of application, particularly, in the range of 0.1 to 0.2% by mass with respect to the total mass at the time of application. When the heat tolerance or drying tolerance improving agent, the salt tolerance improving agent, or the activity improving agent for plants of the present invention is, for example, in a liquid dosage form, the agriculturally effective amount of the acetic acid, etc. is, for example, in the range of 0.01 to 0.5% by volume with respect to the total volume at the time of application, usually in the range of 0.05 to 0.5% by volume with respect to the total volume at the time of application, typically in the range of 0.075 to 0.25% by volume with respect to the total volume at the time of application, more typically in the range of 0.09 to 0.2% by volume with respect to the total mass at the time of application, particularly, in the range of 0.1 to 0.2% by volume with respect to the total mass at the time of application.

In each aspect of the present invention, the malic acid, etc. is contained or applied in an agriculturally effective amount. In each aspect of the present invention, the agriculturally effective amount of the malic acid, etc. is, for example, in the range of 0.01 to 0.5% by mass with respect to the total mass at the time of application, usually in the range of 0.05 to 0.5% by mass with respect to the total mass at the time of application, typically in the range of 0.075 to 0.25% by mass with respect to the total mass at the time of application, more typically in the range of 0.09 to 0.2% by mass with respect to the total mass at the time of application, particularly, in the range of 0.1 to 0.2% by mass with respect to the total mass at the time of application. When the heat tolerance or drying tolerance improving agent, the salt tolerance improving agent, or the activity improving agent for plants of the present invention is, for example, in a liquid dosage form, the agriculturally effective amount of the malic acid, etc. is, for example, in the range of 0.01 to 0.5% by volume with respect to the total volume at the time of application, usually in the range of 0.05 to 0.5% by volume with respect to the total volume at the time of application, typically in the range of 0.075 to 0.25% by volume with respect to the total volume at the time of application, more typically in the range of 0.09 to 0.2% by volume with respect to the total mass at the time of application, particularly, in the range of 0.1 to 0.2% by volume with respect to the total mass at the time of application.

In plant cultivation, conditions under which the heat tolerance or drying tolerance improving agent, the salt tolerance improving agent, or the activity improving agent for plants of the present invention is applied to the plant, a material for application to the plant, or soil, a medium, or a culture solution where the plant grows can be appropriately set on the basis of the growth status of the plant. As a result, the growth of the plant can be stably controlled while heat tolerance or drying tolerance, salt tolerance, or activity of the plant can be improved.

The present invention also relates to a method for controlling the growth of a plant, comprising:
obtaining one or more pieces of information on the growth of the plant (hereinafter, also referred to as an "information obtainment step"); and
on the basis of the obtained one or more pieces of information, determining conditions under which the heat tolerance or drying tolerance improving agent, the salt tolerance improving agent, the activity improving agent for plants of the present invention is applied to the plant, a material for application to the plant, or soil, a medium, or a culture solution where the plant grows (hereinafter, also referred to as an "application condition determination step").

Examples of the one or more pieces of information on the growth of the plant to be obtained in the information obtainment step can include, but are not particularly limited to, various pieces of information on growth promoting effects such as the elongation of leaves and stems or roots, increase in the number of leaves, the promotion of flowering or fructification, increase in the number of flowers or fruits, increase in plant weight or crop yield, greening, and the promotion of tillering, and various pieces of information on unfavorable influence on plant growth, such as inability to grow (firing), poor growth (e.g., the whitening or yellowing of the whole plant or a portion thereof (e.g., leaves or flowers), decrease in root length or decrease in the number of leaves, or lodging), decrease in growth rate, and decrease in plant weight or crop yield, under heat stress or drying stress, or salt stress conditions or without these conditions.

The growth status of the plant can be evaluated by obtaining the one or more pieces of information listed above.

The conditions for applying the heat tolerance or drying tolerance improving agent, the salt tolerance improving agent, or the activity improving agent for plants of the present invention to be determined in the application condition determination step are appropriately set such that heat tolerance or drying tolerance, salt tolerance, or activity of the plant can be improved by carrying out the application. Examples of the conditions to be determined in this step include, but are not particularly limited to, one or more conditions selected from the group consisting of the composition, pH, amount applied and timing of application of the heat tolerance or drying tolerance improving agent, the salt tolerance improving agent, or the activity improving agent for plants of the present invention, and the content of the acetic acid, etc. and the malic acid, etc. contained as an active ingredient.

Specific values of these conditions can be appropriately set from the ranges listed in the present specification.

In the method for controlling the growth of a plant according to the present invention, the number and order of the information obtainment step and the application condition determination step are not particularly limited. For example, the information obtainment step and the application condition determination step may be carried out once each in this order; the information obtainment step, the application condition determination step, and subsequently another round of the information obtainment step may be carried out in this order; and the information obtainment step and the application condition determination step may be repetitively carried out a plurality of times in combination, such as a first round of the information obtainment step, a first round of the application condition determination step, a second round of the information obtainment step, and a second round of the application condition determination step.

In the present specification, the application of the heat tolerance or drying tolerance improving agent, the salt tolerance improving agent, or the activity improving agent for plants of the present invention may be interpreted as the application of acetic acid, etc. and malic acid, etc. The application of acetic acid, etc. and malic acid, etc. can be carried out by a method described as "use of acetic acid, etc. and malic acid, etc. in combination" in the present specification.

The present invention may also relate to a method for improving the heat tolerance or drying tolerance, salt tolerance, or activity of a plant by applying thereto acetic acid, etc. and malic acid, etc.

The present invention may further relate to acetic acid, etc. and malic acid for improving the heat tolerance or drying tolerance, salt tolerance, or activity of a plant.

### Examples

Hereinafter, the present invention will be described further specifically with reference to Examples. However, the technical scope of the present invention is not limited by these Examples.

### <Heat drying tolerance test 1>

In a simply prepared incubator, stem lettuce *(Lactuca sativa* L. of the family *Compositae)* was budded and then raised for 3 weeks under conditions involving a temperature of 22°C and a humidity of 40 to 50% in a vinyl pot. During the cultivation period, water supply was performed by irrigation with 100 mL/3 days of water per plant.

A plant that was irrigated, at its root, with 50 mL of water and allowed to absorb it over 24 hours was assigned to water treatment group 1, and a plant that was irrigated, at its root, with 50 mL of a 10 mM aqueous acetic acid solution and allowed to absorb it over 24 hours was assigned to acetic acid treatment group 1.

Then, the vinyl pot was transferred to another tray, which was then left standing in an incubator under conditions involving continuous light, a temperature of 42°C, and a humidity of 40% without water supply.

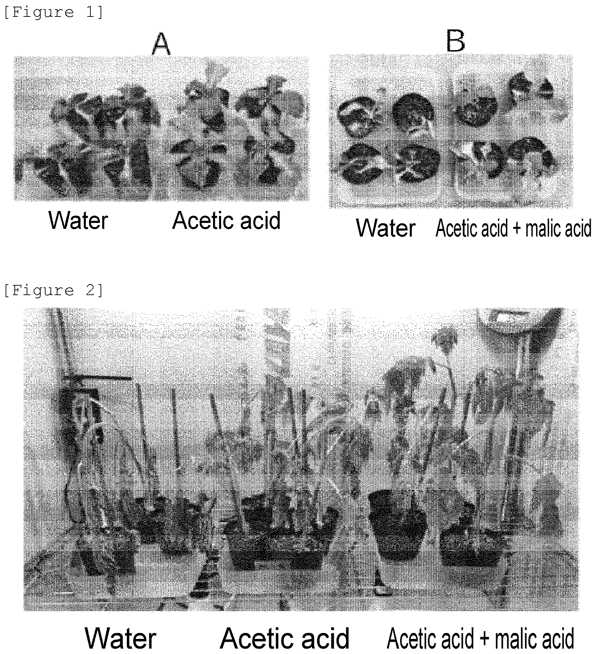

The vinyl pot was taken out of the incubator, and the state of the stem lettuce was observed to measure the survival rate of the stem lettuce. The survival rate was calculated by counting the number of stem lettuce seedlings that were not killed by firing. The results obtained after still standing for 5 days are shown in Figure 1A.

Both the survival rates of the water treatment group 1 and the acetic acid treatment group 1 thus left standing for 5 days were 100%. However, as shown in Figure 1A, the water treatment group 1 evidently wilted more severely, albeit without firing, than the acetic acid treatment group 1. The survival rates after still standing for 7 days were 100% for the acetic acid treatment group 1 and 0% for the water treatment group 1.

### <Heat drying tolerance test 2>

The survival rate of stem lettuce was measured in the same way as in the heat drying tolerance test 1. In this test, conditions involved a temperature of 50°C and a humidity of 10%.

A plant that was irrigated, at its root, with 50 mL of water and allowed to absorb it over 24 hours was assigned to water treatment group 2, and a plant that was irrigated, at its root, with 50 mL of an aqueous solution containing 10 mM acetic acid and 10 mM malic acid and allowed to absorb it over 24 hours was assigned to treatment group 2.

The vinyl pot thus left standing for 3 days was taken out of the incubator, and the state of the stem lettuce was observed to measure the survival rate of the stem lettuce. The survival rate was 100% for the treatment group 2 and 0% for the water treatment group 2. The results are shown in Figure 1B.

In the tests given below, stem lettuce and tomato were allowed to grow under continuous light, whereas conditions involved approximately 5000 lx except that illuminance in an incubator under conditions involving a temperature of 42°C was set to approximately 3300 lx.

From the comparison between the results about the water treatment group 1 and the water treatment group 2, it is evident that the conditions of the water treatment group 2 are more severe growth environment conditions for plants because the plants in the water treatment group 2 were completely killed by firing (see Figures 1A and 1B).

From the results about the acetic acid treatment group 1, it is evident that the acetic acid treatment potentiated the ability of plants to tolerate drying at a high temperature. From the results about the treatment group 2, which was raised under more severe environment conditions, it is evident that the application of acetic acid supplemented with malic acid further potentiated the ability of acetic acid to tolerate drying at a high temperature

### <Heat drying tolerance test 3>

In a simply prepared incubator, tomato (breed: Momotaro, *Solanum lycopersicum* of the family *Solanaceae)* was budded and then raised for 3 weeks under conditions involving a temperature of 22°C and a humidity of 40 to 50% in a vinyl pot. During the cultivation period, water supply was performed by irrigation with 100 mL/3 days of water per plant.

A plant that was irrigated, at its root, with 50 mL of water and allowed to absorb it over 24 hours was assigned to water treatment group 3, and a plant that was irrigated, at its root, with 50 mL of a 20 mM aqueous acetic acid solution and allowed to absorb it over 24 hours was assigned to acetic acid treatment group 3. Also, a plant that was irrigated, at its root, with 50 mL of an aqueous solution containing 10 mM acetic acid and 10 mM malic acid and allowed to absorb it over 24 hours was assigned to treatment group 3.

Then, the vinyl pot was transferred to another tray, which was then left standing in an incubator under conditions involving continuous light, a temperature of 50°C, and a humidity of 10% without water supply.

The vinyl pot thus left standing for 3 days or 4 days was taken out of the incubator, and the state of the tomato was observed to measure the survival rate of the tomato. The survival rate was calculated by counting the number of stem lettuce seedlings that were not killed by firing. The results obtained after still standing for 3 days are shown in Figure 2.

The survival rate after still standing for 3 days was 0% for the water treatment group 3 and 100% for both the acetic acid treatment group 3 and the treatment group 3. The survival rate after still standing for 4 days was 0% for the water treatment group 3 and the acetic acid treatment group 3 and 100% for the treatment group 3.

It was found that the ability to tolerate drying at a high temperature was more potentiated in the acetic acid + malic acid treatment group 3, the acetic acid treatment group 3, and the water treatment group 3 in this order.

### <Activation test 1>

Peperomia *(Peperomia albovittata* of the family *Piperaceae)* obtained by division around the same time among samples was purchased and then raised for 2 days under conditions involving a temperature of 22°C and a humidity of 40 to 50% in a simply prepared incubator. During the cultivation period, water supply was performed by irrigation with 50 mL/day of water per plant.

A plant that was irrigated, at its root, with 50 mL of water and allowed to absorb it over 24 hours was assigned to water treatment group 1; a plant that was irrigated, at its root, with 50 mL of a 20 mM aqueous acetic acid solution and allowed to absorb it over 24 hours was assigned to acetic acid treatment group 1; and a plant that was irrigated, at its root, with 50 mL of an aqueous solution containing 20 mM acetic acid and 0.075 µM malic acid and allowed to absorb it over 24 hours was assigned to treatment group 1.

Then, the vinyl pot was transferred to another tray, which was then left standing in an incubator under conditions involving continuous light, a temperature of 22°C, and a humidity of 40%.

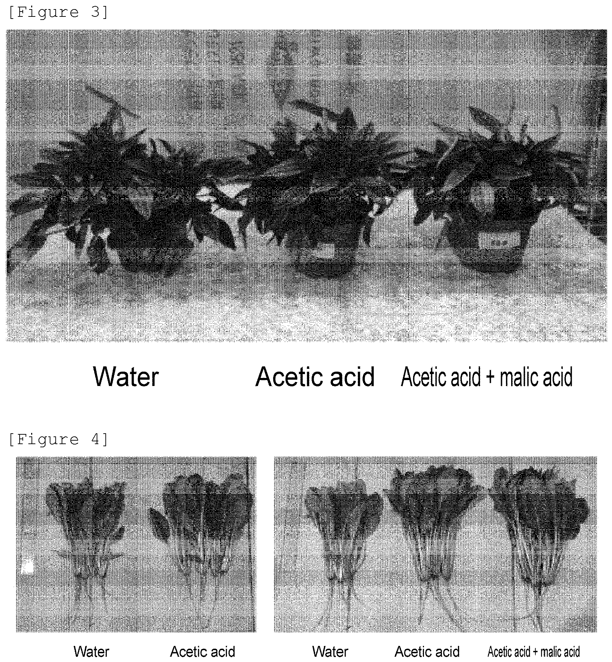

The vinyl pot thus left standing for 3 weeks was taken out of the incubator, and the state of the tomato was observed to count the number of flower buds formed at the early flowering stage and the number of flower buds after flowering that grew into a length of 3 cm or larger in each peperomia individual. The results obtained after still standing for 3 days are shown in Figure 3.

The number of flower buds formed before flowering was 3 in the water treatment group 1 thus left standing for 3 weeks, and the number of flower buds formed before flowering was 5 in the acetic acid treatment group 1. The number of flower buds after flowering that grew into 3 cm or larger was 0 in both the water treatment group 1 and the acetic acid treatment group 1. By contrast, in the treatment group 1, the number of flower buds formed before flowering was 12, and the number of flower buds after flowering that grew into 3 cm or larger was 6.

It was found that the induction and growth of flower buds were potentiated in the acetic acid + malic acid treatment group 1 compared with each of the acetic acid treatment group 1 and the water treatment group 1.

### <Activation test 2>

Spinach (*Spinacia oleracea* of the family *Amaranthaceae*) budded from seeds direct-seeded in a farm field in a plastic greenhouse was used.

During the test period, each plant was watered twice a day at 6 a.m. and 4 p.m. from an automatically controlled irrigation system.

2 weeks after budding, a seedling having three to five true leaves was irrigated, at its root, with 50 mL of each testing solution given below. 2 weeks and 4 weeks after the initial irrigation treatment, the plant was irrigated again with 50 mL of the testing solution (irrigated a total of 3 times).

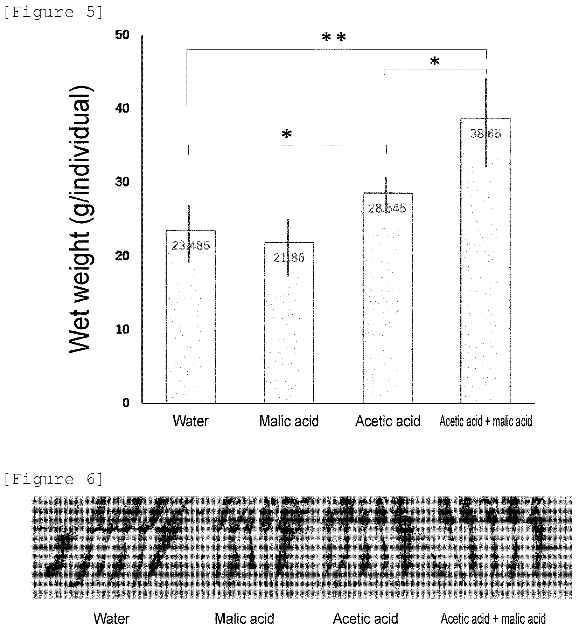

45 days after the first irrigation treatment, plant individuals were harvested, and the wet weights of 20 individuals per test group were measured. The average weight and standard deviation of the plants in each test group were calculated. The significant difference in wet weight among the test groups was calculated by Welch's T test (Welch test). The results are shown in Figures 4 and 5. The results of the Welch test in these and following drawings mean *: p < 0.05 and **: p < 0.01.

The testing solutions used are as described below. Testing solutions prepared in the same way were also used in activation tests 3 to 6 given below.
Water treatment group 2: sterile distilled water
Malic acid treatment group 2: 0.24% (w/w) aqueous malic acid solution
Acetic acid treatment group 2: 0.06% (w/w) aqueous acetic acid solution
Treatment group 2: aqueous solution containing 0.24% (w/w) malic acid and 0.06% (w/w) acetic acid

Each solution was adjusted to pH = 6.0 using KOH.

No significant difference in wet weight was confirmed between the water treatment group 2 and the malic acid treatment group 2, whereas significant increase of 122% in wet weight (p < 0.05) was confirmed between the water treatment group 2 and the acetic acid treatment group 2. More significant increase of 165% in wet weight (p < 0.01) was confirmed between the water treatment group 2 and the acetic acid + malic acid treatment group 2.

### <Activation test 3>

Carrot (*Daucus carota* subsp. *sativus* of the family *Apiaceae*) budded from seeds direct-seeded in a farm field was used.

During the test period, each plant was watered by natural rainfall.

2 weeks after budding, a seedling having four to five true leaves was irrigated, at its root, with 50 mL of each testing solution. 3 weeks and 6 weeks after the initial irrigation treatment, the plant was irrigated again with 50 mL of the testing solution (irrigated a total of 3 times).

90 days after the first irrigation treatment, plant individuals were harvested, and aerial parts (leaf parts) were cut off to prepare edible root parts. Then, the wet weights of 100 individuals per test group were measured. Top 10% and bottom 10% of the data were removed, and the average weight and standard deviation of the plants in each test group were calculated as to the remaining 80 individuals. The significant difference in wet weight among the test groups was calculated by the Welch test.

No significant difference in wet weight was confirmed among the water treatment group 3, the acetic acid treatment group 3 and the malic acid treatment group 3, whereas the significant difference in wet root weight (p < 0.01) after harvest was confirmed between the water treatment group 3 and the acetic acid + malic acid treatment group 3 and between the acetic acid treatment group 3 and the acetic acid + malic acid treatment group 3.

### <Activation test 4>

20 branches per test group were collected from aromaticus (*Plectranthus amboinicus* of the family *Lamiaceae*) that was allowed to grow in culture soil in a 20 cm pot, and each cut off to leave approximately 10 leaves from the shoot apex.

The lower part (stem part) of each cutting was dipped in 200 mL of each testing solution and raised for 2 weeks by exposure to natural light in a greenhouse. 2 weeks later, each plant was taken out thereof, and the number of new roots, root elongation and root branches thereof were measured. Their means and standard deviations were determined, and the significant difference among the test groups was calculated by the Welch test.

New roots were confirmed in neither the water treatment group 4 nor the acetic acid treatment group 4, whereas new roots were confirmed in the acetic acid treatment group 4 and the acetic acid + malic acid treatment group 4 by the treatment with each solution. The significant difference in the number of new roots, root length and the number of branches was confirmed between the water treatment group 4 and the acetic acid + malic acid treatment group 4 and between the acetic acid treatment group 4 and the acetic acid + malic acid treatment group 4. Marked new root formation, elongation, and branching were confirmed in the acetic acid + malic acid treatment group 4 compared with the acetic acid treatment group 4.

### <Activation test 5>

On the day before harvest, spinach planted in the ground in a plastic greenhouse was irrigated, at its root, with 100 mL/plant of each testing solution from the surface of the ground using a sprinkling can.

18 hours later, the aerial part was harvested by cutting at the root with a cutter, and 5 bunches were wrapped with a piece of newspaper having the same area (30 cm x 45 cm) among samples.

Four types in total of test groups (20 individuals in total) including all the test groups were placed in a plastic bag (25 cm X c30m), positioned in an upright posture with the mouth of the bag opened, and stored in a refrigerator of 4°C with a humidity of 40%. Timedependent change in the weight of each individual was measured with a weight scale. When the weight of each plant at the start of the test was defined as 100%, change in the weight of each treatment group was calculated on an individual basis. The mean and standard deviation of change in the weights of 20 individuals at each time per group were determined.

No significant difference in the rate of decrease in wet weight was confirmed between the water treatment group 5 and the malic acid treatment group 5, whereas a marked water retaining effect appeared at and after 12 hours from the start of refrigeration in the acetic acid treatment group 5 and the acetic acid + malic acid treatment group 5 compared with the water treatment group 5 and the malic acid treatment group 5.

After the completion of the experiment for 96 hours, 7.1% and 22% reduction preventing effects on decrease in water content were detected in the acetic acid treatment group 5 and the acetic acid + malic acid treatment group 5, respectively, compared with the water treatment group 5. An effect of maintaining the freshness of plants after harvest was shown in the acetic acid treatment group 5 and the acetic acid + malic acid treatment group 5.

### <Activation test 6>

Swamp chrysanthemum (*Leucanthemum paludosum* of the family *Compositae*) raised in a 9 cm pot was used.

Swamp chrysanthemum seedlings that grew through seeding and budding around the same time were raised by providing 100 mL/plant of water every 3 days for 2 weeks. Five seedlings per test group were provided, and each dipped in 200 mL of each testing solution and left for 24 hours so as to absorb the solution from the bottom of the pot. All apical buds of the plants thus treated with the solution were cut off (pinched) with scissors.

After apical bud cutting, the plants were raised by water supply with 200 mL/plant of tap water every 4 days in an outdoor greenhouse. 3 weeks later, the number of new apical buds (flower buds) was measured.

No significant difference in the number of new flower buds was seen between the water treatment group 6 and the malic acid treatment group 6, whereas increase in the number of flower buds was confirmed in the acetic acid treatment group 6 and the acetic acid + malic acid treatment group 6 compared with the water treatment group 6. The significant difference was also confirmed between the acetic acid treatment group 6 and the acetic acid + malic acid treatment group 6.

It was revealed that acetic acid can promote the new formation and formation of apical buds and flower buds and further, use of acetic acid and malic acid together more strongly promotes the new formation and formation of apical buds and flower buds.

## Claims

1. A heat tolerance or drying tolerance improving agent, a salt tolerance improving agent, or an activity improving agent for plants, comprising acetic acid or a salt thereof, or a solvate thereof and malic acid or a salt thereof, or a solvate thereof.

2. A heat tolerance or drying tolerance improving agent, a salt tolerance improving agent, or an activity improving agent for plants, comprising acetic acid or a salt thereof, or a solvate thereof, for use in combination with malic acid or a salt thereof, or a solvate thereof.

3. A heat tolerance or drying tolerance improving agent, a salt tolerance improving agent, or an activity improving agent for plants, comprising malic acid or a salt thereof, or a solvate thereof, for use in combination with acetic acid or a salt thereof, or a solvate thereof.

4. The heat tolerance or drying tolerance improving agent, the salt tolerance improving agent, or the activity improving agent according to any one of claims 1 to 3, further comprising one or more solvents including at least water.

5. The heat tolerance or drying tolerance improving agent, the salt tolerance improving agent, or the activity improving agent according to claim 4, wherein a pH is in the range of 3 to 9.

6. A composition for improving heat tolerance or drying tolerance, salt tolerance, or activity of a plant, comprising acetic acid or a salt thereof, or a solvate thereof and malic acid or a salt thereof, or a solvate thereof.

7. A composition for improving heat tolerance or drying tolerance, salt tolerance, or activity of a plant, comprising acetic acid or a salt thereof, or a solvate thereof, for use in combination with malic acid or a salt thereof, or a solvate thereof.

8. A composition for improving heat tolerance or drying tolerance, salt tolerance, or activity of a plant, comprising malic acid or a salt thereof, or a solvate thereof, for use in combination with acetic acid or a salt thereof, or a solvate thereof.

9. The composition according to any one of claims 6 to 8, further comprising one or more solvents including at least water.

10. The composition according to claim 9, wherein a pH is in the range of 3 to 9.

11. A method for improving heat tolerance or drying tolerance, salt tolerance, or activity of a plant, comprising applying acetic acid or a salt thereof, or a solvate thereof and malic acid or a salt thereof, or a solvate thereof to the plant, a material for application to the plant, or soil, a medium, or a culture solution where the plant grows.

12. A method for controlling growth of a plant, comprising:
obtaining one or more pieces of information on the growth of the plant; and
on the basis of the obtained one or more pieces of information, determining conditions under which the heat tolerance or drying tolerance improving agent, salt tolerance improving agent, or activity improving agent according to any one of claims 1 to 5 or the composition according to any one of claims 6 to 10 is applied to the plant, a material for application to the plant, or soil, a medium, or a culture solution where the plant grows.

13. A method for improving heat tolerance or drying tolerance, salt tolerance, or activity of a plant, comprising using acetic acid or a salt thereof, or a solvate thereof and malic acid or a salt thereof, or a solvate thereof in combination.

14. A method for improving heat tolerance or drying tolerance, salt tolerance, or activity of a plant, comprising using acetic acid or a salt thereof, or a solvate thereof and malic acid or a salt thereof, or a solvate thereof.
